# EUROPÄISCHE PATENTSCHRIFT

(11) **EP 2 858 608 B1**
(45) Veröffentlichungstag und Bekanntmachung des Hinweises auf die Patenterteilung: **16.05.2018**
(21) Anmeldenummer: 13728165.5
(22) Anmeldetag: 07.06.2013
(51) Int. Cl.: A61F 5/02, A43C 11/16

(54) **SPANNVORRICHTUNG FÜR ORTHESEN**
TENSIONING DEVICE FOR ORTHOSES
DISPOSITIF DE SERRAGE POUR ORTHÈSES

(30) Priorität: 08.06.2012 DE 102012011742
(43) Veröffentlichungstag der Anmeldung: 15.04.2015
(73) Patentinhaber: Bauerfeind AG, 07937 Zeulenroda-Triebes (DE)
(72) Erfinder: STIER, Gerald, 07957 Langenwetzendorf (DE)
(74) Vertreter: Wohlfahrt, Jan Günther
(86) Internationale Anmeldenummer: PCT/EP2013/061818
(87) Internationale Veröffentlichungsnummer: WO 2013/182685

(56) Entgegenhaltungen:
- US-A1- 2008 066 272
- US-A1- 2012 004 587
- US-A1- 2012 101 417

## Beschreibung

Die Erfindung betrifft eine neuartige Spannvorrichtung für Orthesen zur Stützung und Funktionserhaltung des menschlichen Körpers, besonders körperteilumgreifende oder körperumgreifende Orthesen, beispielsweise Rückenorthesen. Die Spannvorrichtung erlaubt ein individuelles, segmentweises Anpassen der Stützwirkung durch individuell separat ausgebildete Flaschenzüge mit mindestens einer Umlenkrolle und einer arretierbaren Spule.

Orthesen sind therapeutische Hilfsmittel zur Stabilisierung oder Unterstützung der Bewegungsfunktion von Körperteilen, beispielsweise von Becken und Wirbelsäule. Der Einsatz der Orthesen kann direkt posttraumatisch oder postoperativ oder konservativ erfolgen. Bei der Verwendung werden die Orthesen in der Regel um das Körperteil, beispielsweise um die Hüfte herum, angelegt und so manschettenartig oder gürtelartig geschlossen, dass ein stabilisierender Druck auf die zu stabilisierende Körperregion ausgeübt wird. Beispielsweise bei Lumbalorthesen kann es erforderlich sein, eine bestimmte Wirbelsäulenkrümmung (Lordose) zu fixieren, um weitere Schäden an der Wirbelsäule zu verhindern oder einen postoperativen Zustand zu stabilisieren, um die Heilung zu verbessern.

Bekannte Orthesen, beispielsweise Lumbalorthesengürtel, weisen häufig Spannvorrichtungen auf, mit deren Hilfe die Spannung/der Druck der umgelegten Orthese auf das Körperteil kontrolliert erhöht werden kann. Dazu ist häufig eine Flaschenzugvorrichtung vorgesehen. Diese erstreckt sich bekanntermaßen im Wesentlichen über die gesamte Breite der Orthese mit dem Ziel, die Zugkraft über die gesamte Breite des Orthesengürtels zu vergleichmäßigen. Bekannte, auf Flaschenzügen basierende Spannvorrichtungen weisen dazu ein loses Zugseil auf, das zu einer Seite der Orthese hin verläuft. Es hat sich herausgestellt, dass derart aufgebaute Orthesen bei der Verwendung mit Nachteilen behaftet sind: Durch die durch den bekannten Flaschenzugmechanismus bedingte Vergleichmäßigung der Zugkraft ist eine individuelle Anpassung der Spannung und damit der Stützwirkung auf ein individuelles Behandlungsziel nicht möglich. Gleichzeitig kommt es durch das Spannen bekannter Flaschenzugvorrichtungen über einseitige Zugseile zu einer einseitigen Kraftwirkung an der Orthese und damit zu deren Verziehen oder Verrutschen.

Ein verbessertes Flaschenzugsystem ist aus der DE 10 2010 035 309 A1 bekannt. Dort werden mehrere Flaschenzüge verwendet. Eine besondere Konstruktion von Umlenkrollen und/oder Verankerungspunkten des Zugseils ist dort nicht vorgesehen.

Die US 2012/004587 A1 offenbart eine Orthese mit einem Zugseilsystem, das auf arretierbaren Rollen aufgerollt und die Orthese somit gespannt werden kann.

Die Erfindung hat sich die Aufgabe gestellt, Spannvorrichtungen für Orthesen weiterzuentwickeln und zu verbessern, um die bekannten Nachteile zu vermeiden. Das der Erfindung zugrunde liegende technische Problem liegt in der Bereitstellung einer verbesserten Spannvorrichtung für eine Orthese, die insbesondere in ihrer Stützfunktion besser an die Anforderungen der Therapie oder Prophylaxe angepasst werden kann. Gleichzeitig soll die Vorrichtung leichter und zuverlässiger bedienbar und einstellbar sein; ein Verrutschen oder Verwinden soll vermieden werden. Insbesondere soll das Flaschenzugsystem einer Spannvorrichtung konstruktiv verbessert werden und so ausgestaltet sein, dass es stabil und funktionstüchtig ist und zugleich gut und einfach zu bedienen ist.

Das technische Problem wird vollständig gelöst durch eine Spannvorrichtung für eine Orthese nach Anspruch 1.

Das technische Problem wird insbesondere gelöst durch eine Spannvorrichtung für eine Orthese, umfassend mindestens einen Flaschenzug, wobei der Flaschenzug mindestens ein Zugseil und mindestens zwei Zugseilumlenkungen aufweist, wobei mindestens eine der mindestens zwei Zugseilumlenkungen als Umlenkrolle ausgestaltet ist, wobei die mindestens eine Umlenkrolle koaxial mit einer arretierbaren Spule gelagert ist und wobei die Spule zum Aufwickeln und Spannen des mindestens einen Zugseils dient, und das mindestens eine Zugseil auf der Spule aufgerollt oder aufgewickelt wird.

Die Spannvorrichtung kann in vorteilhafter Weise an verschiedensten als Orthesen dienenden Manschetten angebracht werden oder angebracht sein, wenn die Manschetten zusätzlich gezielt, insbesondere segmentweise mit Spannung beauflagt werden sollen.

In einer bevorzugten Ausführungsform sind die Umlenkrolle und die Spule Bestandteile eines Spann- und Umlenkelements.

In einer bevorzugten Ausführungsform weist die Spule Rastelemente zum Arretieren der Spule auf.

Der erfindungsgemäße Aufbau eines Flaschenzugs, bei dem mindestens eine Umlenkrolle koaxial mit einer arretierbaren Spule zum Aufwickeln und Spannen eines Zugseils gelagert ist, und diese bevorzugt als Spann- und Umlenkelement ausgebildet sind, ermöglicht einen einfachen, kompakten und leicht zu bedienenden Aufbau des Flaschenzugs und der Zugseilumlenkung. Der Flaschenzug kann dabei in einfacher Weise durch das Aufrollen oder Aufwickeln des Zugseils auf die Spule gespannt werden. Durch die vorhandenen Rastelemente kann sichergestellt werden, dass ohne aktives Lösen der Rastelemente die Spule sich nicht wieder abrollen kann, so dass das Zugseil gespannt bleibt. Das Spann- und Umlenkelement kombiniert in vorteilhafter Weise drei Funktionen, nämlich das Umlenken des Zugseils, das Spannen des Zugseils und somit des Flaschenzugs und das Aufwickeln des überschüssigen Zugseils.

In einer bevorzugten Ausführungsform weist das Spann- und Umlenkelement zusätzlich mindestens ein Rastelement auf. In einer bevorzugten Ausführungsform umfasst das Rastelement mindestens einen federnd gelagerten Zahn, beispielsweise zwei, drei oder mehr federnd gelagerte Zähne. Besonders bevorzugt umfasst das Rastelement zwei sich gegenüber liegende federnd gelagerte Zähne.

Bevorzugt weist die Spule einen Zahnkranz auf, in den die federnd gelagerten Zähne eingreifen können. Wenn die federnd gelagerten Zähne in den Zahnkranz der Spule eingreifen, wird bevorzugt die Drehbarkeit der Spule in eine Richtung verhindert. Es kann auch vorgesehen sein, dass die Drehbarkeit der Spule in beide Richtungen verhindert wird.

In einer bevorzugten Ausführungsform weist das Spann- und Umlenkelement zusätzlich mindestens ein Gehäuseteil auf. Es können beispielsweise zwei Gehäuseteile vorgesehen sein. Das erste Gehäuseteil kann als Aufnahmeelement für die mindestens eine Umlenkrolle und die Spule und bevorzugt für weitere Elemente, beispielsweise Rastelemente, ausgestaltet sein, das zweite Gehäuseteil kann als Deckel für das erste Gehäuseteil ausgestaltet sein.

In einer bevorzugten Ausführungsform weist das Spann- und Umlenkelement zusätzlich mindestens ein Rastelement, bevorzugt mindestens einen federnd gelagerten Zahn, und mindestens ein Gehäuseteil auf.

In einer besonders bevorzugten Ausführungsform weist das Spann- und Umlenkelement mindestens eine Umlenkrolle, eine arretierbare Spule, zwei federnd gelagerte Zähne, und zwei Gehäuseteile auf, wobei das erste Gehäuseteil als Aufnahmeelement für die mindestens eine Umlenkrolle, die Spule und die zwei federnd gelagerte Zähne ausgestaltet ist und das zweite Gehäuseteil als Deckel für das erste Gehäuseteil ausgestaltet ist.

In einer bevorzugten Ausführungsform ist das erste Gehäuseteil und das als Deckel ausgestaltete zweite Gehäuseteil so ausgebildet, dass sich der Deckel im ersten Gehäuseteil um einen bestimmten Winkel, beispielsweise einen Winkel von 5° bis 90°, insbesondere von etwa 10°, drehen lässt und dass die Drehung des Deckels bewirkt, dass dieser in die federnd gelagerten Zähne eingreift und diese nach außen schiebt, so dass die federnd gelagerten Zähne das Drehen der Spule nicht verhindern. Wird in dieser Ausführungsform der Deckel um den bestimmten Winkel in die andere Richtung gedreht, werden die federnd gelagerten Zähen nicht auseinander gedrückt, sondern greifen in die Zähne der Spule ein, so dass ein Drehen der Spule zumindest in eine Richtung verhindert wird.

In einer bevorzugten Ausführungsform ist das Spann- und Umlenkelement aus Kunststoff. Bevorzugt sind alle Bestandteile des Spann- und Umlenkelements aus Kunststoff.

In einer bevorzugten Ausführungsform weist die Spule einen Angriffpunkt für ein Werkzeug zum Drehen der Spule und Aufwickeln und Spannen des Zugseils auf. Der Angriffspunkt kann beispielsweise ein Kreuzschlitz zum Ansetzen eines Kreuzschlitzschraubendrehers sein. Natürlich sind auch andere dem Fachmann bekannte Angriffspunkte möglich. Der Angriffspunkt kann so ausgestaltet sein, dass er nur mit einem Spezialwerkzeug gedreht werden kann. Damit kann sichergestellt werden, dass das Spannen und Entspannen des Flaschenzugs nur durch einen zuständigen Fachmann, beispielsweise einen Orthopäden, der das entsprechende Werkzeug besitzt, erfolgt, und nicht durch einen Laien, beispielsweise den Patienten selbst. Somit ist das Einstellen einer geeigneten Spannung ermöglicht. Alternativ kann natürlich auch vorgesehen sein, dass die Spannung auch durch den Patienten selbst erfolgen kann, beispielsweise durch die Ausgestaltung des Angriffspunkts als Drehknopf oder Drehscheibe.

In einer bevorzugten Ausführungsform weisen alle Flaschenzüge der Spannvorrichtung mindestens eine Umlenkrolle auf, wobei die Umlenkrolle koaxial mit einer arretierbaren Spule gelagert ist, wobei die Spule zum Aufwickeln und Spannen des mindestens einen Zugseils dient.

In einer erfindungsgemäß bevorzugten Ausgestaltung sind Flaschenzüge mit mehrfacher Umlenkung ausgebildet.

In einer bevorzugten Ausführungsform handelt es sich um eine Spannvorrichtung, enthaltend mehrere separate, unabhängig spannbare Spannsegmente, die jeweils ein erstes Seitenelement an einem ersten Ende und ein zweites Seitenelement an einem gegenüberliegenden zweiten Ende und ein dazwischen angeordnetes Zentralelement aufweisen, wobei erstes und zweites Seitenelement eines Spannsegments über das Zentralelement dieses Spannsegments durch einen zwischen Zentralelement und erstem Seitenelement verlaufenden ersten Flaschenzug oder Flaschenzugpaar und einen zwischen Zentralelement und zweitem Seitenelement verlaufenden zweiten Flaschenzug oder Flaschenzugpaar miteinander verbunden sind.

Besondere Ausgestaltungsformen einer solchen Spannvorrichtung sind in der DE 10 2010 035 309 A1 beschrieben, deren Inhalt in die vorliegende Erfindung mit einbezogen wird. Ein Fachmann kann ohne weiteres die dort offenbarten Ausführungsformen mit mindestens einem erfindungsgemäßen Flaschenzug, bei dem mindestens eine Umlenkrolle koaxial mit einer arretierbaren Spule gelagert ist, wobei die Spule zum Aufwickeln und Spannen des mindestens einen Zugseils dient, kombinieren beziehungsweise mindestens einen solchen Flaschenzug in einer dort offenbarten Ausführungsform einsetzen.

Erfindungsgemäß bevorzugt ist eine erfindungsgemäße Spannvorrichtung für eine Orthese, aufweisend mindestens ein erstes Seitenelement an einem ersten Ende und mindestens ein zweites Seitenelement an einem gegenüberliegenden zweiten Ende und mindestens ein dazwischen angeordnetes Zentralelement, wobei erstes und zweites Seitenelement über das Zentralelement durch mindestens einen zwischen Zentralelement und erstem Seitenelement verlaufenden ersten Flaschenzug und mindestens einen zwischen Zentralelement und zweitem Seitenelement verlaufenden zweiten Flaschenzug miteinander verbunden sind, wobei die Ansatzstellen der Flaschenzüge an das Zentralelement über lösbare mechanische Koppelmittel höhenverstellbar sind.

In einer bevorzugten Ausführungsform ist das erste Seitenelement über mindestens zwei Flaschenzüge mit dem Zentralelement verbunden und das zweite Seitenelement ist über mindestens zwei weitere Flaschenzüge mit dem Zentralelement verbunden, wobei die Ansatzpunkte der mindestens vier Flaschenzüge an das Zentralelement über lösbare mechanische Koppelmittel unabhängig voneinander höhenverstellbar sind.

Es hat sich gezeigt, dass sich die Kraftvektoren einer Orthese, individuell und zielgerichtet auf den Ort von Beschwerden ausgerichtet werden können, wenn Flaschenzüge eingesetzt werden, die individuell an einem Orthesensegment, verstellbar angebracht werden können, so dass die Flaschenzüge dort die größte Spannwirkung an der Stelle des von der Orthese umfassten Körperteils des Patienten entfalten können, wo sie an dem Orthesensegment angebracht werden.

Es hat sich insbesondere gezeigt, dass sich die Kraftvektoren einer Orthese, insbesondere Rückenorthese individuell und zielgerichtet auf den Ort von Rückenbeschwerden ausgerichtet werden können, wenn Flaschenzüge eingesetzt werden, die individuell an einer Rückenplatte, die als Zentralelement dient, höhenverstellbar angebracht werden können, so dass die Flaschenzüge dort die größte Spannwirkung an der Wirbelsäule entfalten können, wo sie an der Rückenplatte angebracht werden. So ist es mit einer erfindungsgemäßen Spannvorrichtung insbesondere möglich bei einer Versorgung eines Patienten mit einer Rückenorthese gezielt und punktgenau bestimmte Wirbelsäulenabschnitte zu belasten oder zu entlasten und somit ein Maximum an therapeutischer Wirksamkeit zu erzielen.

Erfindungsgemäß bevorzugt erfolgt das Anbringen der Flaschenzüge an das Zentralelement über Koppelmittel. Erfindungsgemäß bevorzugt umfasst ein Koppelmittel ein erstes Koppelelement und ein zweites Koppelelement, wobei sich das erste Koppelelement an einem Flaschenzug befindet und das zweite Koppelelement sich an dem Zentralelement befindet.

In einer bevorzugten Ausführungsform befindet sich das erste Koppelelement beim Spann- und Umlenkelement. In einer bevorzugten Ausführungsform ist das erste Koppelelement Bestandteil eines Spann- und Umlenkelements. Bevorzugt kann das erste Koppelelement beispielsweise Bestandteil eines Gehäuseteils des Spann- und Umlenkelements sein, beispielsweise indem das Gehäuseteil und das Koppelelement einstückig aus Kunststoff geformt sind.

Erfindungsgemäß bevorzugt handelt es sich bei dem ersten oder zweiten Koppelelement um ein Einhängelement. Erfindungsgemäß bevorzugt handelt es sich bei dem zweiten oder ersten Koppelelement um eine Aussparung.

Erfindungsgemäß bevorzugt erfolgt das Anbringen der Flaschenzüge an das Zentralelement durch Einhängen von Einhängelementen der Flaschenzüge in Aussparungen des Zentralelements oder durch Einhängen von Einhängelementen des Zentralelements in Aussparungen der Flaschenzüge.

Die Einhängeelemente können beispielsweise Hintergriffelemente oder Rastelemente sein. Die Aussparungen können beispielsweise Löcher sein. Die Löcher können jegliche geeignete Form haben, beispielsweise rund oder oval. Dem Fachmann sind geeignete Formen bekannt.

In einer bevorzugten Ausführungsform sind die lösbaren mechanischen Koppelmittel als Hintergriffelemente oder Rastelemente ausgestaltet.

In einer bevorzugten Ausführungsform sind die Koppelelemente des Zentralelements links und rechts des die Wirbelsäule abdeckenden Bereichs angeordnet.

In einer bevorzugten Ausführungsform sind die Ansatzpunkte des mindestens einen ersten Flaschenzugs und des mindestens einen zweiten Flaschenzug unabhängig voneinander höhenverstellbar. In einer bevorzugten Ausführungsform sind die Ansatzpunkte aller Flaschenzüge der Spannvorrichtung unabhängig voneinander höhenverstellbar.

In einer bevorzugten Ausführungsform ist die Spannwirkung des Spannsegments durch Verringerung des Abstands der Seitenelemente zueinander über die Flaschenzüge erzielbar.

In einer bevorzugten Ausführungsform verlaufen die Zugseile jeweils in einem zwischen Zentralelement und Seitenelementen angeordneten Gitterrahmen.

In besonderer Ausgestaltung der Erfindung werden die Zugseile eines Flaschenzugs in einem Zugseiltunnel geführt. Der Zugseiltunnel erstreckt sich bevorzugt zwischen dem Seitenelement und dem Zentralelement. Der Zugseiltunnel ist bevorzugt aus einem elastischen flexiblen Werkstoff ausgeführt; besonders bevorzugt ist ein elastisches Gestrick. Die Erfindung ermöglicht so eine kompakte und anwenderfreundliche Realisierung der segmentweisen individuellen Flaschenzüge. Ein Inkontaktbringen der Zugseile benachbarter Flaschenzüge und ein Verhaken mit Kleidungsstücken oder anderen Orthesenbestandteilen werden so wirkungsvoll verhindert.

In einer bevorzugten Ausführungsform verlaufen die Zugseile des Flaschenzugs in einem zwischen Seitenelement und Zentralelement verlaufenden Zugseiltunnel aus einem elastischen Werkstoff verlaufen.

In einer bevorzugten Ausführungsform ist die Spannvorrichtung speziell ausgebildet, um über lösbare mechanische Koppelmittel im Bereich der Seitenelemente und/oder des Zentralelements auf eine Gestrickorthese aufrüstbar zu sein.

Die Spannsegmente weisen bevorzugt jeweils ein erstes Seitenelement an einem ersten Ende der Spannvorrichtung und ein zweites Seitenelement an einem gegenüberliegenden zweiten Ende der Spannvorrichtung auf. Dazwischen ist ein bevorzugt mittig zwischen dem ersten und zweiten Seitenelement ein Zentralelement angeordnet. Die Seitenelemente können über das Zentralelement miteinander mechanisch verbunden sein, und zwar über jeweils einen zwischen dem Zentralelement und dem ersten Seitenelement verlaufenden ersten Flaschenzug und einen zwischen dem Zentralelement und dem zweiten Seitenelement verlaufenden zweiten Flaschenzug, und bilden so ein Spannsegment.

Im angelegten Zustand ist die Spannvorrichtung zusammen mit der Orthese um das Körperteil herum gelegt und die beiden Enden der Spannvorrichtung stehen miteinander in kraftschlüssiger Verbindung; die Spannvorrichtung ist wie ein Gürtel um das Körperteil geschlossen. Die Erfindung sieht innerhalb eines einzelnen Spannsegments zu beiden Seiten eines Zentralelements jeweils einen Flaschenzug vor, der sich zu den Seitenelementen hin erstreckt. Die sich gegenüber liegenden Flaschenzüge eines Spannsegments können bei der Benutzung der angelegten Orthese über ein beidseitiges Ziehen der losen Enden ihrer beiden Zugseile in entgegengesetzter Richtung zirkulär gespannt werden. Beim Spannen wird der Abstand zwischen den Seitenelementen und damit den beiden Enden der Spannvorrichtung zueinander zu verkürzt und eine das Körperteil umlaufende zirkuläre Spannung erzeugt. Dadurch wird vorteilhafterweise eine zum Zentralelement symmetrische Krafteinwirkung erreicht. Die zirkuläre Spannung kann so unmittelbar symmetrisch gleichmäßig auf beiden Seiten des Zentralelements wirken, sodass dieses beim Spannen nicht aus seiner Position verschoben wird. Ein Verwinden oder ein Verschieben der Spannvorrichtung oder der mit der Spannvorrichtung verbundenen Orthese beim Spannen wird wirksam verhindert.

In Abkehr vom Stand der Technik sieht die Erfindung also auch bevorzugt vor, mehrere Flaschenzüge vorzugsweise im Wesentlichen parallel zueinander unabhängig in separaten Spannsegmenten anzuordnen. Jeder Flaschenzug greift jeweils ausschließlich an den ihm zugeordneten Zentralelement und Seitenelementen eines Spannsegments an. Jedem einzelnen Spannsegment ist also in dieser Ausgestaltung nur ein einziges individuell einstellbares Paar von Flaschenzügen zugeordnet. Jeder Flaschenzug ermöglicht in Verbindung mit dem ihm gegenüberliegenden, zum anderen Ende hin weisenden Flaschenzug eine segmentweise individuell einstellbare symmetrische zirkuläre Spannwirkung.

Sind in dem einen Kraftansatzpunkt des Spannsegments, sei es an Zentralelement oder dem Seitenelement, mehrere Zugseilumlenkungen vorgesehen, so ist bevorzugt, dass die Umlenkungen als gestapelte Umlenkrollen mit gemeinsamer Achse in dem einzigen Kraftansatzpunkt einen Rollenblock bilden.

In einer besonderen Ausgestaltung sind die Zugseile unmittelbar in das Gestrick einer Orthese eingelegt. Dazu weist das Gestrick der Orthese vorzugsweise tunnelartige Aussparungen oder Laschen auf, die eine getrennte Führung der Zugseile ermöglichen. Zusätzliche Maßnahmen wie der in der vorbezeichneten Ausgestaltung vorgesehene Gitterrahmen zur Beabstandung der Zugseile benachbarter Flaschenzüge erübrigen sich in dieser Ausgestaltung. In der vereinfachten Ausgestaltung einer bereits in die Gestrickorthese integrierten erfindungsgemäßen Spannvorrichtung ist eine hierin beschriebene Auf- und Abrüstbarkeit der Spannvorrichtung auf eine stehende, insbesondere herkömmliche Orthese, besonders Gestrickorthese, nicht erforderlich; die erfindungsgemäße Spannvorrichtung ist vielmehr ein integraler Bestandteil einer entsprechenden neuartigen hierin beschriebenen erfindungsgemäßen Orthese.

In einer besonderen Variante sind jeweils die losen Enden der Zugseile der Flaschenzüge benachbarter Spannsegmente in einen gemeinsamen Angriffspunkt geführt und darin zueinander beabstandet fixiert.

Seitenelement und/oder Zentralelement weisen bevorzugt Verbindungsmittel auf, um mit der Orthese, gegebenenfalls lösbar, verbunden zu werden. Die Erfindung erlaubt so die Auf- beziehungsweise Abrüstung der Spannvorrichtung auch auf eine bestehende Orthese. Besonders ist die bestehende Orthese eine herkömmliche Gestrickorthese, besonders eine Staborthese, die aufgrund ihrer Eigenelastizität um das Köperteil herumgeführt und in an sich bekannter Weise über Laschen geschlossen wird.

Die Orthese kann insbesondere eine einen Körperteil umfassende oder umgreifende Manschette sein. Die Orthese kann beispielsweise eine Rückenorthese, eine Knieorthese, eine Handorthese oder eine Armorthese sein. Bevorzugt ist eine Rückenorthese

Im Falle einer Rückenorthese kann die erfindungsgemäße Spannvorrichtung im Bereich der Rückenseite und der Hüftseite auf die Orthese aufgerüstet werden.

Die erfindungsgemäße Spannvorrichtung erlaubt eine individuelle Anpassung der Spannkraft einer Orthese über einen weiten Bereich. Da die stabilisierende beziehungsweise fixierende Wirkung der Orthese im Wesentlichen vollständig durch die Spannvorrichtung selbst übernommen wird, braucht eine darunterliegende elastische in an sich bekannter Weise ausgebildete Gestrickorthese selbst keine vollständige Spann- oder Stützwirkung mehr ausüben. Es kann daher vorteilhafterweise ein Gestrick verwendet werden, das über einen großen Umfangsbereich, im Falle einer Rückenorthese beispielsweise sowohl bei schmaler Taille als auch bei großem Bauchbeziehungsweise Brustumfang, eingesetzt werden kann, ohne dass jeweils individuell an die Körpergröße angepasste Gestrickorthesen bereitgestellt werden müssen. Die Anpassung an den Körperumfang kann dabei jeweils bevorzugt ausschließlich über die Einstellung der Flaschenzüge, also durch Aufwickeln oder Abwickeln des Zugseils auf die beziehungsweise von der Spule und dementsprechenden Spannen oder Entspannen, vorgenommen werden.

Die vorliegende Erfindung betrifft auch einen Flaschenzug zur Verwendung in einer Spannvorrichtung für eine Orthese, wobei der Flaschenzug mindestens ein Zugseil und mindestens zwei Zugseilumlenkungen aufweist, wobei mindestens eine der mindestens zwei Zugseilumlenkungen als Umlenkrolle ausgestaltet ist, wobei die mindestens eine Umlenkrolle koaxial mit einer arretierbaren Spule gelagert ist und wobei die Spule zum Aufwickeln und Spannen des mindestens einen Zugseils dient.

Die vorliegende Erfindung betrifft auch ein Spann- und Umlenkelement zur Verwendung in einer Spannvorrichtung für eine Orthese oder in einem Flaschenzug in einer Spannvorrichtung für eine Orthese, wobei das Spann- und Umlenkelement eine Umlenkrolle und eine arretierbare Spule aufweist, wobei die mindestens eine Umlenkrolle koaxial mit einer arretierbaren Spule gelagert ist und wobei die Spule zum Aufwickeln und Spannen des mindestens einen Zugseils dient. Bevorzugte Ausführungsformen eines erfindungsgemäßen Spann- und Umlenkelements sind der entsprechenden Beschreibung einer erfindungsgemäßen Spannvorrichtung zu enthnehmen.

Die vorliegende Erfindung betrifft auch eine Orthese, enthaltend eine erfindungsgemäße Spannvorrichtung.

Die vorliegende Erfindung betrifft auch die Verwendung einer erfindungsgemäßen Spannvorrichtung zur segmentweisen und positionsgenauen Steuerung der Spann- oder Stützfunktion einer Orthese.

Weiter bevorzugte Ausführungsformen der Erfindung ergeben sich aus den Unteransprüchen und den Nebenansprüchen.

Weitere Ausgestaltungsformen der vorliegenden Erfindung kann der Fachmann ohne weiteres von der DE 10 2010 035 309 A1 ableiten.

Die Erfindung wird durch die nachfolgenden Figuren und Figurenbeschreibungen näher illustriert, ohne dass diese beschränkend zu verstehen wären.

Es zeigen
- Figur 1: einen Ausschnitt einer Ausführungsform einer erfindungsgemäßen Spannvorrichtung;
- Figur 2: eine Explosionszeichnung einer Ausführungsform eines erfindungsgemäßen Spann- und Umlenkelements;
- Figur 3: eine Explosionszeichnung einer weiteren Ausführungsform eines erfindungsgemäßen Spannund Umlenkelements;

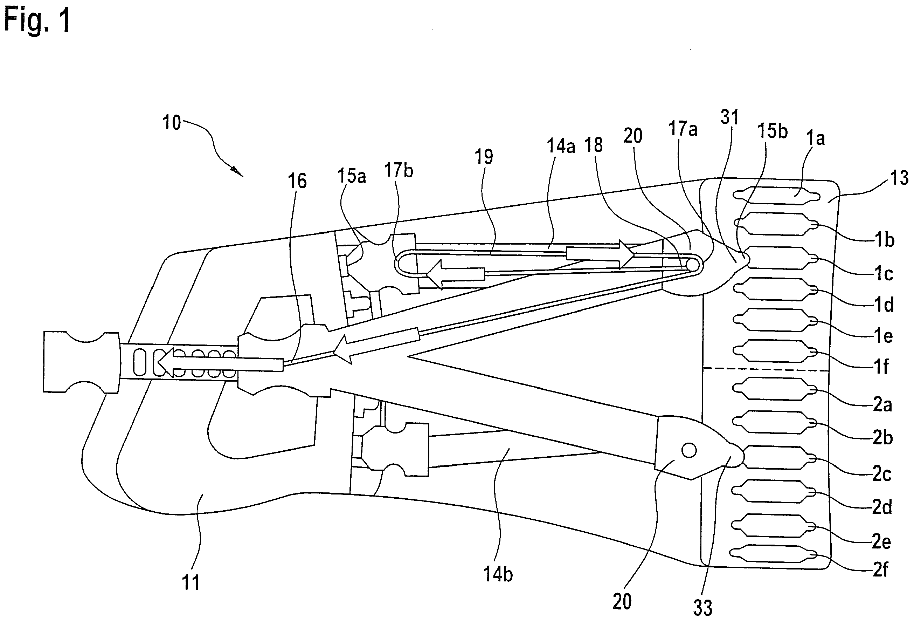

Figur 1 zeigt schematisch einen Ausschnitt aus einer weiteren Ausgestaltung der erfindungsgemäßen Spannvorrichtung. Die Ansicht zeigt aus Gründen der Übersichtlichkeit nur ein Seitenelement (11) und das Zentralelement (13). Das zweite Seitenelement würde in gleicher Weise wie das erste Seitenelement an die andere Hälfte des Zentralelements anschließen, nur gespiegelt. Das Seitenelement (11) ist mit dem Zentralelement (13) über zwei Zugseilantriebe (14a, 14b) in Form von Flaschenzügen mechanisch verbunden. Die Flaschenzüge (14a, 14b) weisen jeweils Zugseilumlenkungen (17a, 17b) und Zugseilverankerungen (18) für ein Zugseil (19) auf, um jeweils einen mehrfachen Flaschenzug zu bilden. Pro Spannsegment sind die Flaschenzüge (14a, 14b) so in jeweils einem Kraftansatzpunkt (15a, 15b) an Zentralelement (13) und Seitenelement (11) verankert. Die Flaschenzüge (14a, 14b) weisen jeweils lose Seilenden (16) auf.

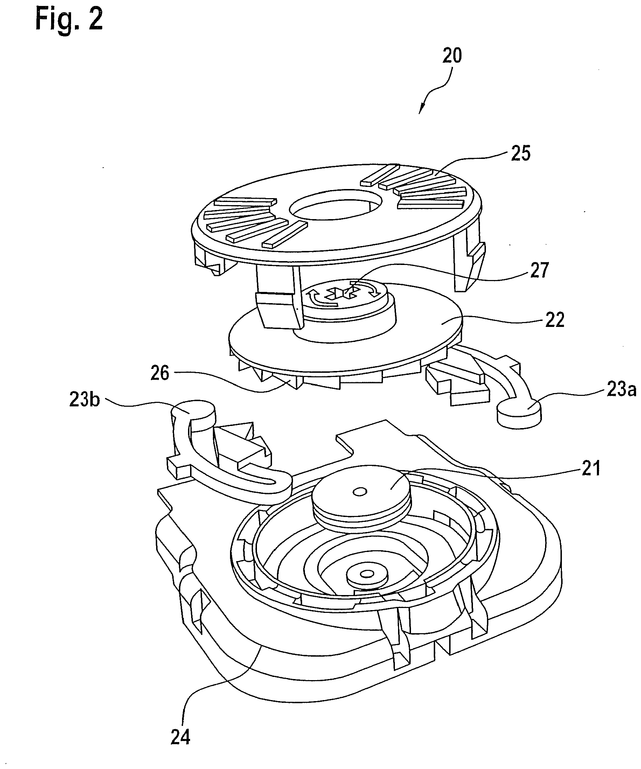

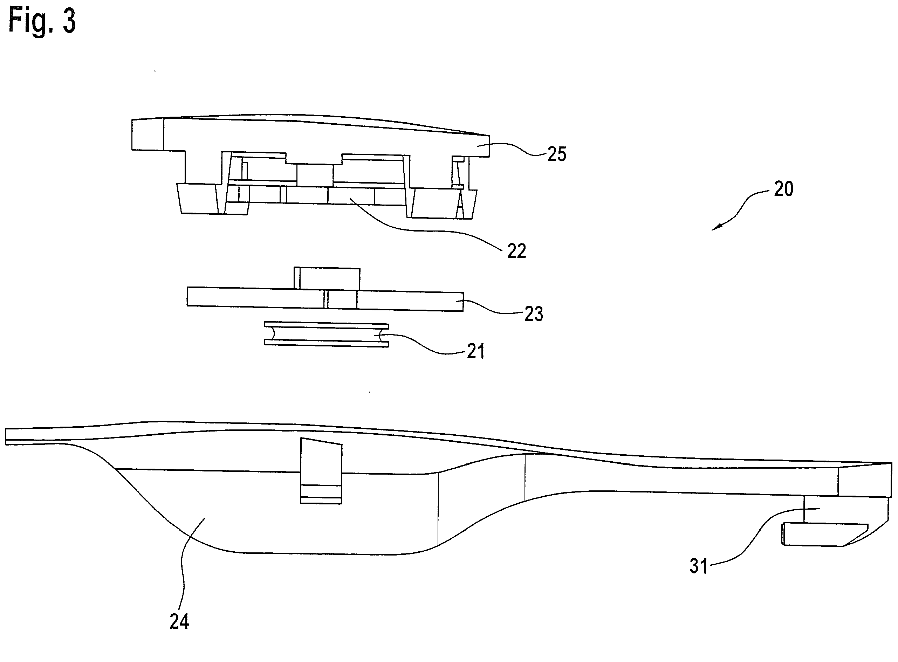

Die Zugseilumlenkung (17a) ist erfindungsgemäß als Umlenkrolle ausgestaltet, die Zugseilverankerung (18) ist erfindungsgemäß als Spule ausgebildet, mit der das dort befindliche Ende des Zugseils (19) aufgewickelt werden kann. Die Umlenkrolle (17a) und die Spule (18) sind koaxial gelagert und sind Bestandteil eines Spann- und Umlenkelements (20). Ein solches erfindungsgemäßes Umlenkelement ist in den Figuren 2 und 3 näher gezeigt.

Die vorliegende erfindungsgemäße Ausführungsform zeichnet sich auch dadurch aus, dass die Ansatzstellen, also Kraftansatzpunkte der Flaschenzüge (14a, 14b) an das Zentralelement (13) der Spannsegmente über lösbare mechanische Kopplungsmittel höhenverstellbar sind. Die mechanischen Kopplungsmittel umfassen in den Zentralelementen (13, 23) befindliche Langlöcher (1a bis 1f, 2a bis 2fb) mit verkleinerten Enden und Kopplungselemente (31, 33), die Bestandteil des Spann- und Umlenkelements (20) sind, und die mit einem Hintergriffelement in die Langlöcher (1a bis 1f, 2a bis 2fb) des Zentralelements (13) eingehakt werden können. Das Kopplungselement (31) kann in eines der oberen Langlöcher (1a bis 1f) eingehängt werden. Das Kopplungselement (33) kann in eines der unteren Langlöcher (2a bis 2f) eingehängt werden. Somit wird in erfindungsgemäßer und vorteilhafter Weise erreicht, dass die Kraftansatzpunkte der einzelnen Flaschenzüge (14a, 14b) an das Zentralelement (13) höhenverstellbar sind und somit in einem gewünschten Bereich wirken können. Ein Fachmann wird ohne weiteres erkennen, das ein zweites Seitenelement auf gleiche Weise mit dem Zentralelement (13) verbunden werden kann.

Figur 2 zeigt eine Explosionszeichnung einer Ausführungsform eines erfindungsgemäßen Spann- und Umlenkelements (20). Dieses umfasst eine Zugseilumlenkung in Form einer Umlenkrolle (21), eine Spule (22) zum Aufwickeln und Spannen eines Zugseil, zwei Rastelemente in Form von federnd gelagerten Zähnen (23a, 23b), einen ersten Gehäuseteil (24) und einen als Deckel ausgebildeten zweiten Gehäuseteil (25). Die Umlenkrolle (21) und die Spule (22) sind koaxial gelagert. Die Spule (22) weist weiterhin eine Verzahnung (26) auf, die in die Zahnfedern (23a, 23b) eingreift. Weiterhin weist die Spule (22) einen Angriffspunkt (27) für ein Werkzeug in Form eines Kreuzschlitzes auf.

Der Deckel (25) kann im ersten Gehäuseteil (24) um einen bestimmten Winkel, beispielsweise 10°, gedreht werden. Der Deckel (25) kann dadurch in die Zahnfedern (23a, 23b) eingreifen und diese nach außen schieben. Dadurch wird der Freilauf entriegelt und die Spule (22) kann gegen den Uhrzeigersinn gedreht werden.

Wird der Deckel (25) wieder um den bestimmten Winkel zurückgedreht, so greifen die federnden Zähne (23a, 23b) in die Verzahnung (26) ein. Mit einem Kreuzschlitzschraubendreher kann die Spule (22) im Uhrzeigersinn gedreht werden, so dass ein nicht gezeigtes Zugseil auf die Spule (22) aufgerollt und somit gespannt werden kann. Ein Wiederabrollen des Zugseils ist in dieser Position nicht möglich, da dies durch das Eingreifen der Zahnfedern (23a, 23b) in die Zähne (26) der Spule (22) verhindert wird.

Figur 3 zeigt eine weitere Ausführungsform eines erfindungsgemäßen Spann- und Umlenkelements (20) in einer Explosionszeichnung. Es sind wieder die Umlenkrolle (21), die Spule (22), ein Rastelement in Form eines federnd gelagerten Zahns (23), ein erstes Gehäuseteil (24) und ein als Deckel ausgeführtes zweites Gehäuseteil (25) zu sehen. Die Funktionsweise ist die gleiche wie die des in Figur 2 gezeigten Spann- und Umlenkelements.

Das erste Gehäuseteil (24) ist vorliegend so ausgestaltet, dass es ein Kopplungselement (31) aufweist, das in ein Zentralelement einer erfindungsgemäßen Spannvorrichtung eingekoppelt werden kann. Das Kopplungselement (31) ist vorliegend als Hintergriffelement ausgestaltet, das in Langlöcher eines Zentralelementes der Spannvorrichtung eingehakt werden kann.

## Patentansprüche

1. Spannvorrichtung für eine Orthese, umfassend mindestens einen Flaschenzug (14a, 14b), wobei der mindestens eine Flaschenzug (14,a 14b) mindestens ein Zugseil (19) und mindestens zwei Zugseilumlenkungen (17a, 17b) aufweist, wobei mindestens eine der mindestens zwei Zugseilumlenkungen (17a, 17b) als Umlenkrolle ausgestaltet ist, **dadurch gekennzeichnet,**
**dass** die mindestens eine Umlenkrolle (21) koaxial mit einer arretierbaren Spule (22) gelagert ist, wobei die Spule (22) zum Aufwickeln und Spannen des mindestens einen Zugseils (19) dient und das mindestens eine Zugseil (19) auf der Spule (22) aufgerollt oder aufgewickelt wird.

2. Spannvorrichtung nach Anspruch 1, wobei die Umlenkrolle (21) und die Spule (22) Bestandteile eines Spann- und Umlenkelements (20) sind.

3. Spannvorrichtung nach einem der vorhergehenden Ansprüche, wobei die Spule (22) eine Verzahnung (26) zum Arretieren der Spule (22) aufweist.

4. Spannvorrichtung nach einem der Ansprüche 2 bis 3, wobei das Spann- und Umlenkelement (20) zusätzlich mindestens ein Rastelement (23a, 23b), bevorzugt mindestens einen federnd gelagerten Zahn, und mindestens ein Gehäuseteil (24, 25) aufweist.

5. Spannvorrichtung nach einem der vorhergehenden Ansprüche, wobei die Spule (22) einen Angriffpunkt (27) für ein Werkzeug zum Drehen der Spule (22) und Aufwickeln und Spannen des Zugseils (19) aufweist.

6. Spannvorrichtung nach einem der vorhergehenden Ansprüche, enthaltend mehrere separate, unabhängig spannbare Spannsegmente (10), die jeweils ein erstes Seitenelement (11) an einem ersten Ende und ein zweites Seitenelement an einem gegenüberliegenden zweiten Ende und ein dazwischen angeordnetes Zentralelement (13) aufweisen, wobei erstes und zweites Seitenelement (11) eines Spannsegments (10) über das Zentralelement (13) dieses Spannsegments durch einen zwischen Zentralelement (13) und erstem Seitenelement (11) verlaufenden ersten Flaschenzug (14) oder Flaschenzugpaar und einen zwischen Zentralelement (13) und zweitem Seitenelement verlaufenden zweiten Flaschenzug oder Flaschenzugpaar miteinander verbunden sind.

7. Spannvorrichtung nach Anspruch 6, wobei die Spannwirkung des Spannsegments (10) durch Verringerung des Abstands der Seitenelemente (11) zueinander über die Flaschenzüge (14) erzielbar ist.

8. Spannvorrichtung nach einem der vorstehenden Ansprüche, wobei die Zugseile (19) jeweils in einem zwischen Zentralelement (13) und Seitenelementen (11,12) angeordneten Gitterrahmen verlaufen.

9. Spannvorrichtung nach einem der vorstehenden Ansprüche, wobei die Zugseile (19) des Flaschenzugs (14) in einem zwischen Seitenelement (11) und Zentralelement (13) verlaufenden Zugseiltunnel aus einem elastischen Werkstoff verlaufen.

10. Spannvorrichtung nach einem der vorstehenden Ansprüche, speziell ausgebildet, um über lösbare mechanische Koppelmittel im Bereich der Seitenelemente (11) und/oder des Zentralelements (13) auf eine Gestrickorthese aufrüstbar zu sein.

11. Spannvorrichtung nach einem der vorstehenden Ansprüche, wobei alle Flaschenzüge der Spannvorrichtung mindestens eine Umlenkrolle aufweisen, wobei die Umlenkrolle koaxial mit einer arretierbaren Spule gelagert ist, wobei die Spule zum Aufwickeln und Spannen des mindestens einen Zugseils dient.

12. Spannvorrichtung nach einem der vorstehenden Ansprüche für eine Orthese, wobei die Orthese eine Rückenorthese, eine Knieorthese, eine Handorthese oder eine Armorthese ist.

13. Orthese, enthaltend die Spannvorrichtung nach einem der Ansprüche 1 bis 11.

14. Orthese nach Anspruch 13, wobei die Orthese eine Rückenorthese, eine Knieorthese, eine Handorthese oder eine Armorthese ist.

## Claims

1. Tensioning device for an orthosis, comprising at least one pulley assembly (14a, 14b), the at least one pulley assembly (14a, 14b) having at least one pulling cable (19) and at least two pulling cable deflections (17a, 17b), at least one of the at least two pulling cable deflections (17a, 17b) being designed as a deflection roller, **characterised in**
**that** the at least one deflection roller (21) is coaxially mounted with a lockable reel (22), the reel (22) being used to wind up and tension the at least one pulling cable (19) and the at least one pulling cable (19) is rolled up or wind up onto the reel.

2. Tensioning device according to claim 1, wherein the deflection roller (21) and the reel (22) form part of a tensioning and deflecting element (20).

3. Tensioning device according to any one of the previous claims, wherein the reel (22) comprises a gear tooth system (26) for locking the reel (22).

4. Tensioning device according to any one of claims 2 to 3, wherein the tensioning and deflecting element (20) additionally comprises at least one detent element (23a, 23b), preferably at least one spring mounted tooth, and at least one housing part (24, 25).

5. Tensioning device according to any one of the previous claims, wherein the reel (22) has a point of application (27) for a tool for rotating the reel (22) and for winding up and tensioning the pulling cable (19).

6. Tensioning device according to any one of the previous claims, has a plurality of separate, independently tensible tensioning segments (10), each having a first side element (11) on a first end and a second side element on an opposite second end, and a central element (13) arranged in between, wherein the first and second side elements (11) of a tensioning segment (10) are connected to one another via the central element (13) of this tensioning segment by a first pulley assembly (14), or pulley assembly pair, extending between the central element (13) and the first side element (11), and a second pulley assembly, or pulley assembly pair, extending between the central element (13) and the second side element.

7. Tensioning device according to claim 6, wherein the tensioning effect of the tensioning segment (10) can be achieved by decreasing the distance of the side elements (11) to one other by means of the pulley assemblies (14).

8. Tensioning device according to any one of the previous claims, wherein the pulling cables (19) in each case run in a grid frame that is arranged between the central element (13) and the side elements (11, 12).

9. Tensioning device according to any one of the previous claims, wherein the pulling cables (19) of the pulley assembly (14) run in a pulling cable tunnel made of an elastic material, which runs between the side element (11) and central element (13).

10. Tensioning device according to any one of the previous claims, which is specifically designed to be added to a knitted orthosis by way of releasable mechanical coupling means in the region of the side elements (11) and/or of the central element (13).

11. Tensioning device according to any one of the previous claims, wherein all pulley assemblies of the tensioning device have at least one deflection roller, wherein the deflection roller is mounted coaxially with a lockable reel, wherein the reel is used to wind up and tension the at least one pulling cable.

12. Tensioning device, according to any one of the previous claims for an orthosis, wherein the orthosis is a back orthosis, a knee orthosis, a hand orthosis or an arm orthosis.

13. Orthosis, containing the tensioning device according to any one of claims 1 to 11.

14. Orthosis according to claim 13, wherein the orthosis is a back orthosis, a knee orthosis, a hand orthosis or an arm orthosis.

## Revendications

1. Dispositif de serrage pour une orthèse, comportant au moins un palan (14a, 14b), dans lequel l'au moins un palan (14a, 14b) comporte au moins un câble de traction (19) et au moins deux renvois de câble de traction (17a, 17b), au moins un des au moins deux renvois de câble de traction (17a, 17b) étant configuré comme poulie de renvoi, **caractérisé en ce**
**que** l'au moins une poulie de renvoi (21) est supportée coaxialement avec une bobine (22) arrêtable, la bobine (22) étant utilisée pour enrouler et serrer au moins un câble de traction (19) et l'au moins un câble de traction (19) étant enroulé ou bobiné sur la bobine (22).

2. Dispositif de serrage selon la revendication 1, dans lequel la poulie de renvoi (21) et la bobine (22) sont des composants d'un élément de serrage et de renvoi (20).

3. Dispositif de serrage selon l'une quelconque des revendications précédentes, dans lequel la bobine (22) comporte une denture (26) pour arrêter la bobine (22).

4. Dispositif de serrage selon l'une quelconque des revendications 2 à 3, dans lequel l'élément de serrage et de renvoi (20) en outre comporte au moins un élément de verrouillage (23a, 23b), de préférence au moins une dent supportée de manière élastique, et au moins une partie de boîtier (24, 25).

5. Dispositif de serrage selon l'une quelconque des revendications précédentes, dans lequel la bobine (22) comporte un point d'attaque (27) pour un outil pour faire tourner la bobine (22) et pour enrouler et serrer le câble de traction (19).

6. Dispositif de serrage selon l'une quelconque des revendications précédentes, contenant plusieurs segments de serrage (10) individuels qui sont indépendamment serrables et respectivement comportent un premier élément latéral (11) au niveau d'une première extrémité et un second élément latéral au niveau d'une seconde extrémité opposée, et un élément central (13) disposé entre ceux-ci, dans lequel le premier et le second élément latéral (11) d'un segment de serrage (10) sont reliés l'un à l'autre, au moyen de l'élément central (13) de ce segment de serrage, par un premier palan (14) ou une première paire de palans s'étendant entre l'élément central (13) et le premier élément latéral (11), et par un seconde palan ou une seconde paire de palans s'étendant entre l'élément central (13) et le second élément latéral.

7. Dispositif de serrage selon la revendication 6, dans lequel l'action de serrage du segment de serrage (10) peut être effectuée en réduisant la distance mutuelle des éléments latéraux (11) au moyen des palans (14).

8. Dispositif de serrage selon l'une quelconque des revendications précédentes, dans lequel les câbles de traction (19) s'étendent respectivement dans un cadre de grille disposé entre l'élément central (13) et les éléments latéraux (11, 12).

9. Dispositif de serrage selon l'une quelconque des revendications précédentes, dans lequel les câbles de traction (19) du palan (14) passent dans un conduit de câble de traction en matière flexible, le conduit s'étendant entre l'élément latéral (11) et l'élément central (13).

10. Dispositif de serrage selon l'une quelconque des revendications précédentes, le dispositif étant spécifiquement configuré pour être réaménagé sur une orthèse tricotée par moyens d'accouplage mécanique au niveau des éléments latéraux (11) et/ou de l'élément central (13).

11. Dispositif de serrage selon l'une quelconque des revendications précédentes, dans lequel toutes les palans du dispositif de serrage comportent au moins une poulie de renvoi, la poulie de renvoi étant supportée coaxialement avec une bobine arrêtable, la bobine étant utilisée pour enrouler et serrer de l'au moins un câble de traction.

12. Dispositif de serrage selon l'une quelconque des revendications précédentes pour une orthèse, l'orthèse étant une orthèse dorsale, une orthèse de genou, une orthèse de main ou une orthèse de bras.

13. Orthèse contenant le dispositif de serrage selon l'une quelconque des revendications précédentes 1 à 11.

14. Orthèse selon la revendication 13, l'orthèse étant une orthèse dorsale, une orthèse de genou, une orthèse de main ou une orthèse de bras.
